**Europäisches Patentamt**

**European Patent Office**

**Office européen des brevets**

(11) Publication number: **0 406 428 B1**

# EUROPEAN PATENT SPECIFICATION

(45) Date of publication of patent specification: **01.03.95**

(51) Int. Cl.[6]: **C07K 5/12**, C07K 7/64, A61K 38/00, C12N 5/00

(21) Application number: **89910207.3**

(22) Date of filing: **08.09.89**

(86) International application number: **PCT/JP89/00926**

(87) International publication number: **WO 90/02751 (22.03.90 90/07)**

(54) **PEPTIDE DERIVATIVES AND THEIR USE.**

(30) Priority: **09.09.88 JP 224552/88**
     **10.03.89 JP 156350/89**

(43) Date of publication of application:
     **09.01.91 Bulletin 91/02**

(45) Publication of the grant of the patent:
     **01.03.95 Bulletin 95/09**

(84) Designated Contracting States:
     **DE FR GB IT**

(56) References cited:
     **WO-A-88/03560**
     **WO-A-89/05150**

     **JOURNAL OF BIOLOGICAL CHEMISTRY, vol. 262, no. 36, 25 December 1987, American Society for Biochemistry and Molecular Biology Inc. (US); M.D. PIERSCHBACHER et al., pp. 17294-17298&NUM;**

(73) Proprietor: **ASAHI GLASS COMPANY LTD.**
     **1-2, Marunouchi 2-chome**
     **Chiyoda-ku**
     **Tokyo 100 (JP)**

(72) Inventor: **OHBA, Masataka**
     **21-3, Kitaterao 7-chome**
     **Tsurumi-ku**
     **Yokohama-shi**
     **Kanagawa-ken 230 (JP)**
     Inventor: **KUMAGAI, Hiromichi**
     **19-15, Shibuya 1-chome**
     **Shibuya-ku**
     **Tokyo 150 (JP)**
     Inventor: **HARIE, Shunsaku**
     **33-3, Maruyama 1-chome**
     **Isogo-ku**
     **Yokohama-shi**
     **Kanagawa-ken 235 (JP)**
     Inventor: **UCHIDA, Keiichi**
     **23-13, Shibokuhoncho 1-chome**
     **Miyamae-ku**
     **Kawasaki-shi**
     **Kanagawa-ken 213 (JP)**

(74) Representative: **Wächtershäuser, Günter, Prof. Dr.**
**Patentanwalt**
**Tal 29**
**D-80331 München (DE)**

**Description**

TECHNICAL FIELD

This invention relates to a novel synthetic cyclic peptide or peptide derivatives consisting of salts of such synthetic cyclic peptide, as well as to an adhesion inhibitor of animal cells, containing therein, as the effective component, such synthetic cyclic peptide or peptide derivatives.

BACKGROUND ART

As a factor which pertains to adhesiveness of animal cells to extracellular matrices, there have been known fibronectine and vitronectine. These celllar adhesive factors possess their adhesive portion expressed by -Arg-Gly-Asp-. As the consequence, those compounds having the tripeptide residual group same as this adhesive portion inhibit cell-adhesion due to the cellular adhesive factor. In other words, this cell-adhesion inhibiting factor, because of its being bonded to a portion where the cell-adhesion factor is bonded, inhibits any later bonding of the cell-adhesion. Such cell-adhesion inhibiting factor has been known in the form of, for example, Gly-Arg-Gly-Asp-Ser-Pro. Besides this cell-adhesion inhibiting factor has been used as a reagent for researches related to adhesiveness of animal cells, it is also expected to be used as a drug for inhibiting transfer of cancer cells (i.e., inhibiting adhesion and fixation of the cancer cells at a place of their transfer).

Since the heretofore known cell-adhesion inhibiting factors are in the form of linear peptide, its particular steric structure was difficult to exist in a stabilized condition in a solution, on account of which its inhibiting effect, in some cases, could not be exhibited sufficiently. Also, due to presence of the amino end-group and the carboxyl end-group, the conventional adhesion inhibiting factors were readily subjected to hydrolyze by aminopeptidase, carboxypeptidase, and other enzymes, hence they were unstable in a liquid where such enzymes are present.

With a view to improving the structural stability of the cell-adhesion inhibiting factor, there has been known a compound obtained by synthesizing polypeptide having a cysteine residual group, besides the above mentioned tripeptide residual group, and then cyclizing this polypeptide with disulfide bonding (vide: a thesis by M. D. Pierschbadhen et al. in "Journal of Biological Chemistry", Vol. 262, pp. 17294-17298 (1987)). It should, however, be pointed out that even such cell-adhesion inhibiting factor containing therein the disulfide bonding is not still capable of solving the abovementioned problem to a satisfactory extent.

DISCLOSURE OF THE INVENTION

The present invention is concerned with novel peptide derivatives which have solved the abovementioned problem, and drugs having the cell-adhesion inhibiting activity, containing therein such peptide derivatives as the effective component.

Peptide derivatives composed of a synthetic cyclic peptide to be represented by the following formula [I], or salts of the synthetic cyclic peptide:

$$\boxed{\text{Arg-Gly-Asp-R}} \qquad \cdots \ [\text{I}]$$

(where: R denotes an amino acid residual group or oligo-polypeptide residual group).

A drug for inhibiting adhesion of animal cells, which contains therein, as the effective component, a synthetic cyclic peptide to be represented by the following formula [I], or peptide derivatives consisting of salts of the synthetic cyclic peptide:

$$\boxed{\text{Arg-Gly-Asp-R}} \qquad \cdots \ [\text{I}]$$

(where: R denotes an amino acid residual group or oligo-polypeptide residual group).

3

In the present invention, amino acid is meant not only by $\alpha$-amino acid, but also by other amino acids (such as $\beta$-amino acid, $\gamma$-amino acid, and so forth). Appropriate amino acids other than $\alpha$-amino acid are those represented by $H_2N(CH_2)_mCOOH$ (where: m is an integer of 2 or above). Examples of such amino acids are: 3-aminopropionic acid, 4-aminobutanoic acid, 5-aminopentanoic acid, 6-aminocaproic acid, 7-aminopentanoic acid, 8-aminocaprylic acid, and so forth. Preferably, m is an integer of 8 or below. Also, $\alpha$-amino acid may be not only L-amino acid, but also D-amino acid or D,L-amino acid. Of these various amino acids, $\alpha$-amino acid is a preferred one, and L-amino acid is particularly preferable (in the ensuing description, the term "amino acid" is meant by this "L-amino acid", unless otherwise specified). The amino acid residual group is meant by those residual groups after removal of one hydrogen atom from the amino group, and the hydroxy group from the carboxylic group.

In the present invention, "oligo-polypeptide in R refers to one, wherein two or more amino acids as mentioned above are connected by the peptide bond. Preferred number of the amino acid residual group is 16 or less. As the amino acid, $\alpha$-amino acid is preferred. In particular, it is preferred that the entire amino acid residual groups in oligo~polypeptide be $\alpha$-amino acid, although a part of the amino acid residual group may be other residual groups than $\alpha$-amino acid such as the abovementioned $H_2N(CH_2)mCOOH$, $\beta$-amino acid, etc., or D-amino acid residual group. The number of amino acid residual group in oligo~polypeptide should preferably be in a range of from 2 to 11. By the term "oligo-polypeptide residual group", it is meant those residual groups after one hydrogen atom has been removed from the amino group to the side of the amino acid end-group, and the hydroxy group from the carboxylic group to the side of the carboxyl end-group.

In order for the synthetic cyclic peptide according to the present invention to exhibit its cell-adhesion inhibiting effect to be described later, a peptide block of -Ary-Gly-Asp- is required. However, the cyclic peptide having this peptide block alone is not sufficient to exhibit the expected effect, in addition to which at least one amino acid residual group is required to be present. More preferably, at least one $\alpha$-amino acid residual group be present before and after this peptide block (i.e., at both sides of the amino end-group and the carboxy end-group). In particular, it is preferred that the glysine residual group be present at the side of the amino end-group (in other words, the carboxy end-group of R is -Gly-).

Such synthetic cyclic peptide will be represented by the following formula [II]:

$$\boxed{\text{—Arg-Gly-Asp-R}^1\text{-Gly—}} \qquad \cdots[\text{II}]$$

In the above formula [II], $R^1$ denotes a portion other than the glycine residual group of R, wherein the side of the carboxy end-group is the glycine residual group. Preferably, this $R^1$ should be the amino acid residual group, or the oligopeptide residual group having 10 or below of the amino acid residual group.

Further, it is preferred that the carboxyl side of the abovementioned essential peptide block in the form of -Ary-Gly-Asp- contains therein the serine residual group or the asparagine residual group. The subsequent second amino acid residual group should preferably be the proline residual group or the glycine residual group. Moreover, the third amino acid residual group should preferably be the alanine residual group. Accordingly, more preferable R should be oligo-polypeptide to be represented by the following formula [III]:

$$\text{-Ser(or Asn)—[—Pro(or Gly)—][—Ala ]—[—R}^2\text{]} \quad \cdots[\text{III}]$$

In the above formula [III], the three brackets ([ ]) denote the residual groups which should preferably be present (but not indispensable as the case may be), and $R^2$ represents the oligo-polyamino acid residual group or the amino acid residual group, after the concrete amino acid residual group shown in the formula [III] have been removed from R. A particularly preferred R is the oligo~polypeptide residual group to be represented by the following formula [IV]:

$$\text{-Ser(or Asn)- Pro—[—Ala—][—R}^3\text{—]—Gly-} \qquad \cdots[\text{IV}]$$

In the above formula [IV], the two brackets ([ ]) denote the residual groups which should preferably be present (but not indispensable as the case may be), and $R^3$ should preferably be the amino acid residual

4

group, or the oligopeptide residual group having the amino acid residual group of from 2 to 7. There are less limitations to the kind of $R^3$ or the amino acid residual group in $R^3$, a part of which may be the D-amino acid residual group or those residual groups other than $\alpha$-amino acid.

The cyclic peptide represented by the formula [I] is of such a structure that the carboxyl end-group of R and the amino end-group of arginine are connected by the peptide bonding. It should, however, be understood that this cyclic peptide represented by the formula [I] does not show the route for its synthesis. That is to say, this cyclic peptide can be synthesized not only by a method of synthesizing Arg-Gly-Asp-R, followed by cyclization of the same, but also by a method of cyclization by forming the peptide bonding portion at other arbitrary positions. For example, the cyclization can be formed not only between Arg-Gly, Gly-Asp or Asp-R may but also at an arbitrary peptide bonding portion in R. In the following, concrete examples of the cyclic peptide according to the present invention will be listed, without imposing any limitation to the scope of the present invention.

By the way, the $\alpha$-amino residual group in the below-listed cyclic peptides is represented by a single letter.

1. ┌───────────────┐
   └─R─G─D─G─┘

2. R—G—D—S

3. R—G—D—S—G

4. R—G—D—S—P

5. R—G—D—S—P—G

6. R—G—D—S—P—A

7. R—G—D—S—P—A—G

8. R—G—D—S—P—A—T—G

9. R—G—D—S—P—A—V—T—G

10. R—G—D—S—P—A—A—V—T—G

11. R—G—D—V

12. R—G—D—D

13. R—G—D—A

14. R—G—D—I

15. └─R──G──D──P─┘

16. └─R──G──D──L─┘

17. └─R──G──D──K─┘

18. └─R──G──D──N─┘

19. └─R──G──D──T─┘

20. └─R──G──D──N──G─┘

21. └─R──G──D──V──T─┘

22. └─R──G──D──N──P──G─┘

23. └─R──G──D──A──D─┘

24. └─R──G──D──N──P──A──G─┘

25. └─R──G──D──P──D─┘

26. └─R──G──D──K──I─┘

As the salts of the abovementioned peptide, there may be exemplified: organic acid salts or inorganic acid salts of peptide with acetic acid, tartaric acid, citric acid, trifluoroacetic acid, methanesulfonic acid, hydrochloric acid, sulfuric acid, nitric acid, phosphoric acid, and so forth; alkali metal salts of peptide with sodium, potassium, and so on; alkaline earth metal salts of peptide with calcium salt, and so forth; and other salts of peptide with inorganic bases and organic bases such as organic amines like ammonium, ethanolamine, triethylamine, dicyclohexylamine, and so forth.

Cyclic peptide according to the present invention can be synthesized by any ordinary peptide synthesizing methods. As described in the foregoing, the cyclization is carried out at the stage of the cyclization reaction to take place after synthesis of the linear peptide, wherein it is done at a portion by forming an arbitrary peptide bonding between the adjacent amino acid residual groups.

The abovementioned synthetic cyclic peptide according to the present invention is effective as the drug for inhibiting adhesion of the animal cells. As the animal cells, there may be exemplified typically the mammal cells, in addition to which there may further be listed not only ordinary somatic cells, germ cells, but also even cancer cells. It is also effective for inhibiting adhesion of an akaryote such as blood platelet, etc.. Particular animal cells to be the object of the present invention are various cancer cells. The transfer of cancer is ascribable to adhesion of the cancer cells to other cells or the extracellular matrices. Accordingly, the inhibition against adhesion of the cancer cells is considered effective for the prevention of transfer of cancer. It is also considered possible to prevent thrombus, etc. from taking place, if adhesion of the blood platelets to the inner wall of the blood vessel can be hindered. As will be described in various examples to appear later, the synthetic cyclic peptide according to the present invention is excellent not only in its adhesion-inhibiting effect of the animal cells, but also in its stability within the living organism owing to its resistance to hydrolysis by oxygen. Moreover, even if it undergoes hydrolysis, the linear peptide produced by the hydrolysis also possesses its cell-adhesion inhibiting effect to a certain extent, in so far as the abovementioned essential peptide block portion or its vicinity is not subjected to the hydrolysis.

BRIEF DESCRIPTION OF DRAWINGS

Figure 1 is a graphical representation of the results of high performance liquid chromatographic (HPLC) analyses of a cyclic peptide synthesized in accordance with Example 1 (control indicated in solid line) and the same cyclic peptide which was treated with trypsin for 24 hours (in broken line);

Figure 2 is a graphical representation showing the results of the same "HPLC" analyses of a linear peptide for the sake of comparison;

Figure 3 is a graphical representation showing the results of the cell-adhesion hindrance test in accordance with Example 1 C. (in the case of fibronectine coat);

Figure 4 is also a graphical representation showing the test results in the case of vitronectine coat;

Figure 5 is a graphical representation of the result of the "HPLC" analyses showing the decomposition test results of the cyclic peptide synthesized in Example 2;

Figure 6 is also a graphical representation showing the results of the "HPLC" for the sake of comparison; and

Figure 7 is a graphical representation showing the results of the cell-adhesion hindrance test of the cyclic peptide synthesized in accordance with Example 2.

BEST MODE FOR THE CARRYING OUT THE INVENTION

In the following, the present invention will be explained more concretely with reference to actual examples, although the present invention is not limited to these examples alone.

By the way, in the ensuing examples, amino acid, protective group, active group, etc. are, in some cases, expressed by abbreviations based on IUPAC-IUB Commission on Biological Nomenclature as well as conventional abbreviations in this field of technology, some of which will be listed as examples, in the following.

| Ala: | alanine |
|---|---|
| Asn: | asparagine |
| Asp: | asparagic acid |
| Arg: | arginine |
| Gly: | glycine |
| Thr: | threonine |
| Ile: | isoleucine |
| Leu: | leucine |
| Lys: | lysine |
| Pro: | proline |
| Val: | valine |
| Ser: | serine |
| Boc: | t-butoxycarbonyl |
| OBzl: | benzyl ester |
| HOBt: | p-hydroxybenzotriazole |
| OSu: | N-hydroxysuccinimide ester |
| OPac: | phenacyl ester |

WSC•HCl:    1-ethyl-3-(3-dimethylaminopropyl)carbodiimide hydrochloride
TFA:    trifluoroacetic acid
OPFP:    pentafluorophenyl ester
DCC:    dicyclohexylcarbodiimide
DC urea:    cyclohexyl urea
OcHex:    cyclohexyl ester
Tos:    p-toluenesulfonyl group

EXAMPLE 1

A. Synthesis of cyclic peptide ($\overline{\text{R G D S P A G}}$)

(1) Synthesis of BocAla OPac

9.5 g (50 mmol) of BocAla was dissolved in 100 mℓ of methanol, followed by addition of 33 mℓ of 25% (W/V) aqueous solution of $Cs_2CO_3$. This mixture solution was concentrated under a reduced pressure, after which 30 mℓ of toluene was added to this concentrated solution, and then it was solidified under a reduced pressure. This process was repeated for three times to thereby remove the water content in it. Thereafter, 150 mℓ of DMF was added to this solidified substance to dissolve the solid content therein. 10 g (50 m mol) of phenacyl bromide was further added to this dissolved substance, and the whole batch was agitated for one hour at a room temperature. After removal of the precipitated substance (CsBr) by filtration, DMF was removed by fractionation under a reduced pressure. Then, 200 mℓ of ethyl acetate was added this solution, followed by washing the ethyl acetate layer with use of 200 mℓ of water, 200 mℓ of 1N hydrochloric acid (for two times), 200 mℓ of water, 200 mℓ of a 5% aqueous solution of sodium bicarbonate (for two times), and 200 mℓ of water, in the sequence as mentioned. Anhydrous sodium sulfate was added to dessicate the substance, followed by solidifying the same under a reduced pressure, whereupon there was obtained white crystals in a quantity of 13.4 g (143.6 mmol, a rate of yield of 87.2%).

(2) Synthesis of $H_2N$ Ala OPac TFA

13 g (42.3 mmol) of Boc Ala OPac was charged into a flask of 300 mℓ capacity. To this substance, 70 mℓ of trifluoroacetic acid (TFA) was added, and the batch was agitated at room temperature for 20 min., followed by concentrating the same under a reduced pressure. When 300 mℓ of diethyl ether was added to this concentrated liquid, there was deposited white crystals. The white crystals were then collected by filtration, sufficiently washed with diethyl ether, and dried, whereupon 13.2 g (41.1 mmol, a rate of yield of 97.1%) of $H_2N$ Ala OPac•TFA was obtained.

(3) Synthesis of Boc Pro Ala OPac

6.45 g (30 mmol) of BocPro and 9.21 g (30 mmol) of $NH_2$ AlaOPac•TFA were dissolved in 60 mℓ of DMF, to which N-methylmorpholine was added under the ice-cooling conditions, to thereby render the pH value of the solution to be 6. To this solution, 4.6 g of HOBt and 5.8 g of WSC•HCl were added, the pH value of the solution was again rendered to be 6 with use of N-methylmorpholine, and the batch was subjected to agitation overnight. Then, DMF was removed by fractionation under a reduced pressure, after which 100 mℓ of ethyl acetate was added, and the ethyl acetate layer was washed with use of 100 mℓ of water, 100 mℓ of 1N hydrochloric acid (for two times), 100 mℓ of water, 100 mℓ of a 5% aqueous solution of sodium bicarbonate (for two times), and 100 mℓ of water, in the sequence as mentioned. After this washing process, anhydrous sodium sulfate was added to dessicate it.

When the solution was concentrated under a reduced pressure, and then hexane was added to it, there was deposited white crystals, which was collected by filtration and recrystallized from ethyl acetate-hexane, thereby obtaining 6.5 g (16.1 mmol, a rate of yield of 54%) of Boc Pro Ala OPac.

Amino acid analysis values are as follows: Ala 1.0 and Pro 0.96.

(4) <u>Synthesis of Boc Ser(Bzl)•Pro Ala OPac</u>

6.5 g (16 mmol) of Boc Pro Ala OPac was added to 30 mℓ of TFA, and the mixture was agitated for 30 min. at room temperature, after which TFA was removed by fractionation under a reduced pressure to obtain oil. The thus obtained oil was washed for three times with 200 mℓ of diethyl ether, after which diethyl ether was removed under a reduced pressure. 40 mℓ of DMF was added to this oil, and its pH value was adjusted to 6 with addition of N-methylmorpholine under the ice-cooling conditions. Further, there were added 4.72 g (16 mmol) of Boc Ser(Bzl), 2.4 g of HOBt, and 3.1 g of WSC•HCl to this oil, in the order as mentioned, and its pH value was again adjusted to 6 with use of N-methylmorpholine, and the batch was agitated overnight. After removal of DMF under a reduced pressure, the oil was dissolved in 100 mℓ of ethyl acetate, and thereafter, the ethyl acetate layer was washed with 100 mℓ of water, 100 mℓ of 1N hydrochloric acid (for two times), 100 mℓ of water, 100 mℓ of a 5% aqueous solution of sodium bicarbonate (for two times), and 100 mℓ of water, in the order as mentioned. After this washing process, anhydrous sodium sulfate anhydride was added to dessicate it. When ethyl acetate was removed by fractionation under a reduced pressure, there was obtained 7.88 g (13.5 mmol, a rate of yield of 84.4%) of white powder.
The amino acid analyses indicated: Ser 0.90, Pro 0.94, and Ala 1.0.

(5) <u>Synthesis of Boc Asp(OBzl)Ser(Bzl)Pro Ala OPac</u>

7.5 g (13 mmol) of Boc Ser(Bzl)Pro Ala OPac was added to 30 mℓ of TFA, and the batch was agitated for 30 min. at room temperature, after which TFA was removed by fractionation under a reduced pressure to obtain 7.66 g of oil. The thus obtained oil was sufficiently washed with addition of diethyl ether, after which diethyl ether was removed by decantation, which process was repeated for three times. After removal of diethyl ether under a reduced pressure, the oil was dissolved in 30 mℓ of DMF and ice-cooled. Further, there were added to this oil 4.2 g (13 mmol) of Boc Asp(OBzl), 2 g of HOBt, and 3 g of WSC•HCl, in the order as menttoned, and its pH value was adjusted to 6 with use of N-methylmorpholine, and the batch was agitated overnight. The oil obtained by removal of DMF by fractionation under a reduced pressure was dissolved in 100 mℓ of ethyl acetate, and then the ethyl acetate layer was washed with 100 mℓ of water, 100 mℓ of 1N hydrochloric acid (for two times), 100 mℓ of water, 100 mℓ of a 5% aqueous solution of sodium bicarbonate (for two times), and 100 mℓ of water, in the order as mentioned. After this washing process, anhydrous sodium sulfate was added to dessicate it. Powder obtained by removing ethyl acetate under a reduced pressure was recrystallized from ethyl acetate-diethyl ether-hexane, whereupon there was obtained 8.86 g (11.3 mmol, a rate of yield of 86.9%) of white powder.
The amino acid analyses indicated: Asp 1.02, Ser 0.84, Pro 0.97, and Ala 1.0.

(6) <u>Synthesis of Boc Arg(Tos)Gly OBzl</u>

11.3 g (26.3 mmol) of Boc Arg(Tos) was dissolved in 50 mℓ of THF and ice-cooled. To this solution, 8.9 g (26.4 mmol) of Gly OBzl•Tos OH, 4.0 g of HOBt, and 5.1 g of WSC•HCl were added, then the pH value of the solution was adjusted to 6 with use of triethylamine, and the batch was agitated overnight. The oil obtained by removing THF by fractionation under a reduced pressure was dissolved in 100 mℓ of ethyl accetate. The ethyl acetate layer was dissolved in 100 ml of water, 100 ml of 1N hydrochloric acid (for two times), 100 ml of water, and the ethyl acetate layer was washed with use of 100 mℓ of water, 100 mℓ of 1N hydrochloric acid (for two times), 100 mℓ of water, 100 mℓ of a 5% aqueous solution of sodium bicarbonate (for two times), 100 mℓ of water, 100 ml of a 5% aqueous solution of sodium bicarbonate (for two times), and 100 ml of water, in the order as mentioned. After this washing process, anhydrous sodium sulfate was added to dessicate it. After removal of ethyl acetate under a reduced pressure, hexane was added, whereupon 13.3 g (22.5 mmol, a rate of yield of 85.5%) of white precipitation was obtained.
The amino acid analyses indicated: Ala 0.99, and Gly 1.0.

(7) <u>Synthesis of Boc Gly Arg(Tos)Gly OBzl</u>

80 mℓ of TFA was added to 13 g (22 mmol) of Boc Arg (Tos)Gly OBzl, and the batch was agitated for 15 min. at room temperature. After this, TFA was removed by fractionation under a reduced pressure, followed by adding diethyl ether to sufficiently wash the precipitated substance. After removal of diethyl ether under a reduced pressure, the precipitated substance was dissolved in 100 mℓ of THF, and then 3.85 g (22 mmol) of Boc Gly, 3.4 g of HOBt, and 4.22 g of WSC•HCl were added to the solution under the ice-cooling condition, while its pH value was adjusted to 6 with addition of trimethylamine, and the batch was agitated overnight. Oil obtained by removal of THF by fractionation under a reduced pressure was dissolved in 100 mℓ of ethyl acetate, and then the ethyl acetate layer was washed with 100 mℓ of water, 100 mℓ of 1N hydrochloric acid (for two times), 100 mℓ of water, 100 mℓ of a 5% aqueous solution of sodium bicarbonate (for two times), and 100 mℓ of water, in the order as mentioned. After this washing process, anhydrous sodium sulfate was added to dessicate it. Thereafter, when ethyl acetate was removed by fractionation under a reduced pressure, 13.5 g (21.3 mmol, a rate of yield of 96.8%) of white powder was obtained.

The amino acid analyses indicated: Ala 0.99, and Gly 2.0.

(8) Synthesis of Boc Gly Arg(Tos)Gly OH

13.5 g (21.3 mmol) of Boc Gly Arg(Tos)Gly OBzl was dissolved in 100 mℓ of methanol, to which there were added 50 mℓ of acetic acid, 20 mℓ of water, and 5% palladium carbon, followed by passing hydrogen through the mixed solution for three hours. After removal of 5% palladium carbon by filtration, and of the solvent by fractionation under a reduced pressure, the residual substance was dissolved in 100 mℓ of ethyl acetate and dried with use of magnesium sulfate. After filtration, ethyl acetate was removed by fractionation under a reduced pressure, whereupon 9.76 g (19.4 mmol, 91%) of white powder was obtained.

(9) Synthesis of H$_2$N Asp(OBzl)Ser(Bzl)Pro Ala OPac•TFA

50 mℓ of TFA was added to 8 g (10 mmol) of Boc Asp(OBzl) Ser(Bzl)Pro Ala OPac, and the batch was agitated for 30 min. at room temperature. After this, TFA was removed by fractionation under a reduced pressure. Diethyl ether was then added to this residual substance and kept at a temperature of -20°C, whereupon white crystals were deposited. The supernatant liquid was removed by decantation, and the residue was recrystallized from methanol/ethanol. There was obtained an intended product in a quantity of 7.9 g (9.9 mmol, a rate of yield of 99%).

(10) Synthesis of Boc Gly Arg(Tos)Gly Asp(OBzl)Ser(Bzl)Pro Ala OPac

1.5 g (2 mmol) of H$_2$N Asp(OBzl)Ser(Bzl)Pro Ala OPac•TFA was dissolved into 10 mℓ of DMF, to which 1.2 g (2 mmol) of Boc Gly Arg(Tos)Gly OH, 0.3 g of HOBt, and 0.5 g of WSC•HCl were added. The pH value of the mixture was adjusted to 6 with addition of N-methylmorpholine, and the batch was then agitated overnight. Oil as obtained by removing DMF by fractionation under a reduced pressure was dissolved in 50 mℓ of ethyl acetate, and then the ethyl acetate layer was washed with 50 mℓ of water, 50 mℓ of 1N hydrochloric acid (for two times), 50 mℓ of water, 50 mℓ of a 5% aqueous solution of sodium bicarbonate (for two times), and 50 mℓ of water, in the order as mentioned. After this washing process, sodium anhydrous sulfate was added to dessicate it. White powder as obtained by removing ethyl acetate by fractionation under a reduced pressure was sufficiently washed with diethyl ether and dried, whereby 1.36 g (1.06 mmol, a rate of yield of 50%) of white powder was obtained.

The amino acid analyses indicated: Gly 2.0, Arg 0.97, Asp 0.98, Ser 0.85, Pro 0.89, and Ala 1.0.

(11) Synthesis of Boc Gly Arg(Tos)Gly Asp(OBzl)Ser(Bzl)Pro Ala OH

1.0 g (0.8 mmol) of Boc Gly Arg(Tos)Gly Asp(OBzl)Ser(Bzl)Pro Ala OPac was dissolved in 30 mℓ of 90% acetic acid, to which 3.3 g (40 mmol) of zinc powder was added under the ice-cooling condition, and the batch was agitated for three hours at 0°C. After removal of zinc powder by filtration, the solvent was eliminated by fractionation under a reduced pressure, followed by addition of 30 mℓ of 1N hydrochloric acid to the residue. This residue was extracted with use of 50 mℓ of ethyl acetate. Then, the ethyl acetate layer was washed with 50 mℓ of 1N hydrochloric acid and then 50 mℓ of water, after which it was dessicated with sodium sulfate anhydride. Ethyl acetate was concentrated under a reduced pressure, followed by addition of diethyl ether to obtain 0.85 g (0.72 mmol, a rate of yield of 90%) white powder.

## (12) Synthesis of ⌐GlyArg(Tos)GlyAsp(OBzl)Ser(Bzl)ProAla⌐

0.85 g (0.72 mmol) of Boc Gly Arg(Tos)Gly Asp(OBzl)Ser(Bzl)ProAla OH was dissolved in 5 mℓ of DMF, to which 0.15 g (1.3 mmol) of HOSu and 0.25 g of WSC•HCl were further added. Then, the pH value of the mixture solution was adjusted to 6 with use of N-methylmorpholine, after which it was agitated overnight. Oil obtained by adding water to the solution, after DMF was removed by fractionation under a reduced pressure, was sufficiently washed, separated from the batch, and dried under a reduced pressure. To this oil, 20 mℓ of TFA was added and the mixture was agitated for ten minutes at room temperature, after which TFA was removed by fractionation under a reduced pressure. White powder deposited by adding diethyl ether was separated by filtration in a quantity of 0.79 g (0.61 mmol, 84%). This white powder was dissolved in 5 mℓ of DMF, and the solution was dripped into pyridine kept at a temperature of 60°C, in 30 minutes, while stirring the same. After the dripping, the batch was kept at 60°C for five hours, after which it was agitated overnight at a temperature of 30°C. Then, pyridine was removed by fractionation under a reduced pressure, and white powder obtained by addition of diethyl ether was re-crystallized from acetone/diethyl ether. 0.61 g (0.57 mmol, 935) of white powder was finally obtained.

## (13) Synthesis of ⌐GlyArgGlyAspSerProAla⌐

1 mℓ of anisole and 1 mℓ of cresol were added to 0.6 g (0.57 mmol) of

⌐GlyArg(Tos)GlyAsp(OBzl)Ser(Bzl)ProAla⌐.

Then, 50mℓ of hydrofluoric acid was added to this mixture, and the batch was agitated for one hour at a temperature of 0°C. After this, these solvents were removed by fractionation under a reduced pressure, followed by adding diethyl ether to thereby obtain white powder by filtration. This white powder was dissolved in 100 mℓ of water, and the solution was subjected to freeze-drying. 0.40 g of such white powder was obtained. The thus obtained white powder was dissolved in 10 mℓ of a 0.1% aqueous solution of TFA, which solution was provided for HPLC using a semi-dispensing ODS column to thereby collect a 10% fraction of acetonitrile. 40 mg of the intended product was obtained by freeze-drying of the fraction.

The amino acid analyses indicated: Gly 1.96, Arg 0.95, Asp 0.97, Ser 0.82, Pro 0.87, and Ala 1.0.

### (14) Synthesis of linear peptide (G R G D S P A)

The product as synthesized in accordance with the process as described in (11) above was de-protected and refined in the same manner as in (13) above, to thereby obtain GlyArgGlyAspSerProAla.

### B. Peptide map

Peptide as synthesized in accordance with the process of (13) above was decomposed with trypsin, from which it was verified that the compound was cyclic peptide.

Peptide was dissolved in 10 mg/mℓ of distilled water. 20 μl of this solution was diluted with addition of 180 μl of a buffer solution (Tris-HCl, 100 mM, pH 8.5). To this diluted solution, 5 μl of a trip solution (1 mg/mℓ 5M HCl, CaCl$_2$ 10 mM) was added, and the mixture was kept at a temperature of 37°C for 24 hours. Then, 10 μl of 2N HCl was added to this mixed solution to complete the reaction. Out of this reaction liquid, 50 μl was provided for high performance liquid chromatography HPLC for its analysis.

Condition for Analysis HP
HPLC: Waters Model 510

Column: YMC-ODS

Detection: $A_{214}$

Elution:     Liquid A - 0.1% aqueous solution of TFA

             Liquid B - 0.1% acetonitrile solution of TFA

             Linear concentration gradient of:

             0% B → 30% B

             (0 min.) (30 min.)

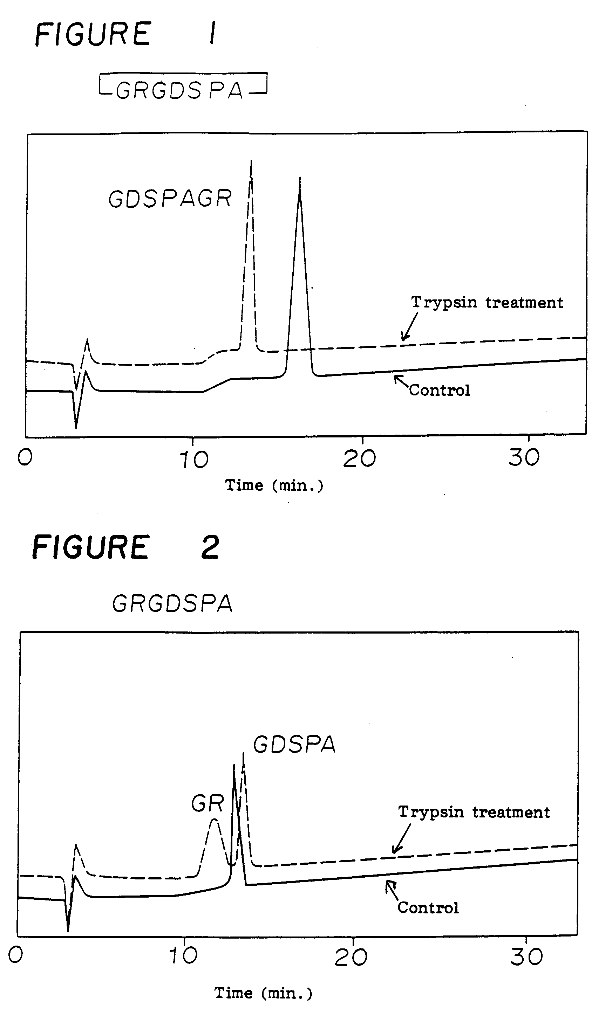

Figure 1 is a graphical representation showing the result of analysis of the cyclic peptide synthesized in accordance with this Example. Also, the linear peptide (GRGDSPA) synthesized in accordance with (14) above was tested under the same conditions as mentioned above. The result of its analysis is shin in Figure 2.

From the results as indicated in Figures 1 and 2, it was verified that peptide obtained from Example 1 (13) was cyclic, and that, in comparison with linear peptide, the cyclic peptide could be opened its ring by treatment with trypsin, but the compound was difficult to undergo further decomposition.

EXAMPLE 2

A.   Synthesis of cyclic peptide (⌐R G D S P A A V T G⌐)

In the same manner as in Example 1 above, cyclic peptide was synthesized. The method for synthesis will be outlined in the following.

(1) Synthesis of Boc Thr(Bzl)GlyoBzl

```
Boc Thr(Bzl)      6.18 g   20 mmol

GlyoBzl·TosOH     6.74 g   20 mmol  DMF   40 mℓ

WSC·HCl           3.84 g            pH   7

HOBt              3.06 g            N-methylmorpholine

                    ↓

BocThr(Bzl)GlyoBzl

          7.32 g (16 mmol) 80%
```

(2) <u>Synthesis of Boc ValThr(Bzl)GlyoBzl</u>

```
    Product of (1) above      6.0 g (13 mmol)

    TFA                       30 mℓ

    Boc Val                   2.8 g (13 mmol)

    WSC·HCl                   2.5 g

    HOBt                      2.0 g      DMF 30 mℓ

                              —          pH 6

                        ↓                N-methylmorpholine



                    BocValThr(Bzl)GlyoBzl

                        6.75 g (12.2 mmol, 94%)
```

(3) <u>Synthesis of Boc Ala Thr(Bzl)GlyoBzl</u>

```
    Product of (2) above      3.4 g (6.1 mmol)

    TFA                       20 mℓ

    Boc Ala                   1.2 g (6.3 mmol)

    WSC·HCl                   1.2 g

    HOBt                      0.94 g   DMF   15 mℓ

                                       pH 6

                        ↓              N-methylmorpholine

                    BocAlaValThr(Bzl)GlyoBzl

                        3.18 g (5.1 mmol, 83.6%)
```

(4) <u>Synthesis of BocAlaValThr(Bzl)GlyOH</u>

2.8 g (4.5 mmol) of the abovementioned product (3) was suspended in 20 mℓ of methanol, and then 9 mℓ of an aqueous solution of 1N NaOH was dripped into the suspension in an ice-bath. After three hours' stirring, 12 mℓ of 1N HCl was added to the mixture, and methanol was removed by fractionation under a reduced pressure, whereupon white crystals were deposited. The crystals were collected by filtration and sufficiently rinsed with water, followed by drying the same. 2.2 g (4.2 mmol, 93%) of the intended product

was obtained.

(5) <u>Synthesis of BocAlaValThr(Bzl)GlyArg(Tos)GlyoBzl</u>

    Product of (4) above 2.1 g (4 mmol)

    H₂NArgGlyoBzl·TFA      2.3 g (4 mmol)

    WSC·HCl                0.6 g


    HOBt              0.8 g    DMF 15 mℓ  pH 6

                      ↓                    N-methylmorpholine

                      Recrystallization from MeOH/diethyl

                      ether

                      3.39 g (3.5 m mol,  87%)


(6) <u>Synthesis of BocAlaValThr(Bel)GlyArg(Tos)GlyOH</u>

    Product of (5) above 3.2 g (3.3 mmol)

    Methanol               20 mℓ

    Acetone                10 mℓ

    1N NaOH                5 mℓ     3 hours' stirring in

                                    ice-bath

                      ↓         ← 5 mℓ of 1N HCl

                      concentrated under a reduced

                      pressure, collected by

                      filtration, rinsed with water,

                      and dried

                      ↓

    2.81 g (3.2 mmol, 97%)

(7) Synthesis of BocAlaValThr(Bzl)GlyArg(Tos)GlyAsp(OBzl) Sen(Bzl)ProAlaOPac

| | |
|---|---|
| Product of (6) above | 1.8 g (2 mmol) |
| H$_2$NAsp(OBzl)Sen(Bzl) | |
| ProAlaOPac·TFA | 1.5 g (2 mmol) |
| [Product of Example 1 (9) above] | |
| HOBt | 0.3 g |
| WSC·HCl | 0.5 g |

10 m$\ell$ of DMF, pH 6 adjusted with N-methylmorpholine

$\downarrow$

white powder precipitated by addition of water after reaction

$\downarrow$

sufficiently washed with water, 1N HCl, water, 5% NaHCO$_3$, and water

$\downarrow$

dried to obtain 2.8 g (1.8 mmol, 90%) of product

(8) Synthesis of BocAlaValThr(Bzl)GlyArg(Tos)GlyAsp(OBzl) Sen(Bzl)ProAlaOH

Product of (7) above 1.8 g (1.16 mmol)

90% acetic acid 40 mℓ, zinc powder 4.5 g

↓

two hours' stirring in ice-bath

↓

filtered to remove zinc powder, followed

by eliminating acetic acid under a reduced

pressure; then addition of 1N HCl to obtain

white precipitation

↓

collected by filtration and sufficiently

rinsed with water to obtain 1.63 g (1.12 mmol,

96%) of product

17

(9) Sysnthesis of

┌─────────────────────────────────────────────────┐
└──AlaValThr(Bzl)GlyArg(Tos)GlyAsp(OBzl)Sen(Bzl)ProAla──┘

Product of (8) above 1.5 g (1.03 mmol)

HOSu          0.12 g

WSC·HCl       0.3 g

              5 mℓ of DMF, pH 6 adjusted with N-

              methylmorpholine

                    ↓

              1.47 g

                    ↓    ← 20 mℓ of TFA

              concentrated under a reduced pressure

                    ↓

after dissolving into 30 mℓ of DMF, solution was dripped into 1ℓ pyridine maintained at 55°C; the mixture solution was reacted for 5 hrs. at 55°C, and overnight at room temperature, after which pyridine was removed by fractionation under a reduced pressure; water was added to deposit white precipitate, which was collected by filtration, rinsed well with water, and dried; 1.61 g (0.98 mmol, at a rate of yield of 95%) of product was obtained.

(10) Synthesis and refining of ⌐AlaValThrGlyArgGlyAspSen-

-ProAla⌐

| | |
|---|---|
| Product of (9) above | 1.61 g (0.98 mmol) |
| HF | 100 mℓ |
| Anisole | 5 mℓ |
| Cresol | 5 mℓ |

↓

HF was removed by fractionation under a reduced pressure for one hour at a temperature of 0°C, after which diethyl ether was added to obtain white precipitate; after filtration, it was dissolved into water and freeze-dried; 1.01 g of freeze-dried powder was obtained.

The refining of the product was done under the same conditions as in Example 1 (13).

B. Peptide map

Under the same conditions as in Example 1, B. above, the cyclic peptide was subjected to test for its decomposition. The conditions for the analyses are as follows.
Conditions for Analysis
    HPLC: Shimadz LC-6A
    Column: YMC ODS
    Detection: $A_{214}$
    Elution:    Liquid A - 0.1% aqueous solution of TFA
                Liquid B - 0.1% acetonitrile solution of TFA
                Curved concentration gradient of:
                5% B → 80% B
                (0 min.) (30 min.) (B curve 3)

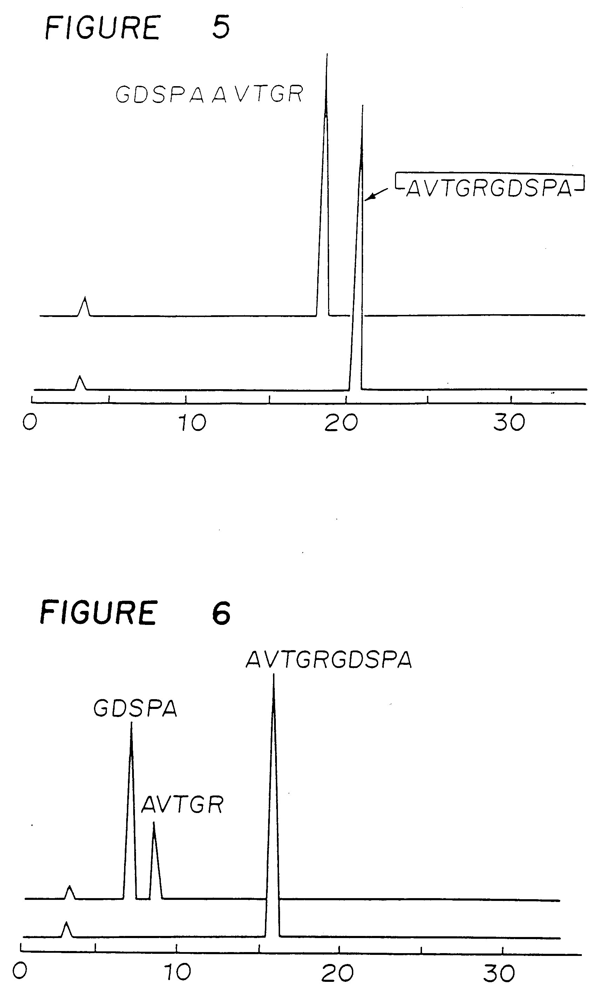

Figure 5 indicates the result of the analysis. Also, corresponding linear peptide (AVTGRGDSPA) was synthesized and subjected to the same test. The results are as shown in Figure 6.

EXAMPLE 3

A. Synthesis of cyclic peptide ( R G D N P A G )

(1) Synthesis of BocAsnOPFP

23.2 g (100 mmol) of BocAsn was dissolved in 200 mℓ of DMF, after which 22.1 g (120 mmol) of pentafluorophenol and 24.7 g (120 mmol) of DCC were added to the mixture under the ice-cooling condition, and the batch was agitated for 30 minutes. After further agitation for one hour at normal temperature, DMF was removed by fractionation under a reduced pressure. The residue was dissolved in ethyl acetate, and filtered to remove DC urea, after which it was rinsed with water and dried by addition of anhydrous sodium sulfate. The residue was further concentrated under a reduced pressure, followed by addition of diethyl ether to crystallize. 16.26 g (a rate of yield of 40.8%) of the product was obtained.

(2) Synthesis of BocAsnProAlaOBzl

20 mℓ of TFA was added to 3.76 g (10 mmol) of BocProAlaOBzl, and the mixture was reacted for 20 minutes at room temperature, followed by removing TFA by fractionation under a reduced pressure. Thereafter, diethyl ether was added to the reaction product to obtain white precipitate, which was then collected by filtration and dried. The thus obtained substance was dissolved in 20 mℓ of DMF, to which 1.5 g of HOBt was added under the ice-cooling condition. Then, the pH value of this solution was adjusted to 6 with use of N-methylmorpholine. To this solution, there was further added 4 g of BocAsnOPFP, and its pH value was again adjusted to 7, followed by stirring the batch overnight. After removal of DMF by fractionation under a reduced pressure, the residue was dissolved in ethyl acetate, the ethyle acetate layer was washed with water, 1N HCl, water, 5% NaHCO$_3$, and water, in the order as mentioned, followed by drying the residual substance with addition of anhydrous sodium sulfate. Then, ethyl acetate was removed by fractionation under a reduced pressure, after which the substance was crystallized from diethyl ether-hexane, thereby obtaining 3.3 g (67.3%) of the intended product.
The amino acid analyses indicated: Asx 1.01, Ala 1.03, and Pro 0.95.

(3) Synthesis of BocAsp(OcHex)AsnProAlaOBzl

20 mℓ of TFA was added to 3 g (6.1 mmol) of BocAsnProAlaOBzl, and the mixture was agitated for 20 minutes at room temperature, followed by removing TFA by fractionation under a reduced pressure. Thereafter, diethyl ether was added to this mixture to obtain white precipitate, which was then collected by filtration and dried. The thus obtained substance was dissolved in 15 mℓ of DMF. Then, the pH value of this solution was adjusted to 6 with use of N-methylmorpholine under the ice-cooling condition. To this solution, there was further added 4.12 g (10 mmol) of BocAsp(OcHex)OSu, and its pH value was again adjusted to 7, followed by stirring the batch overnight. After removal of DMF by fractionation under a reduced pressure, the residue was dissolved in ethyl acetate, to which 1,3-propanediamine was added, and the batch was agitated for one hour at room temperature. Then, the ethyl acetate layer was washed with water, 1N HCl, water, 5% NaHCO$_3$, and water, in the order as mentioned, followed by drying the residual substance with use of anhydrous sodium sulfate. Then, the solvent was concentrated under a reduced pressure, after which the substance was crystallized from ethyl acetate-hexane, thereby obtaining 3.63 g (87%) of the intended product.
The amino acid analyses indicated: Asx 1.98, Ala 0.98, and Pro 1.02.

(4) Synthesis of BocGlyArg(Tos)GlyAsp(OcHex)AsnProAlaOBzl

20 mℓ of TFA was added to 3.43 g (5 mmol) of BocAsp(OcHex)AsnProProAlaOBzl, and the mixture was agitated for 20 minutes at room temperature. After removing TFA by fractionation under a reduced pressure, diethyl ether was added to this mixture to obtain white precipitate, which was then collected by filtration and dried. To the thus obtained substance, there was added 20 mℓ of DMF to dissolve the white precipitate therein. After this, 3 g (4.9 mmol) of BocGlyArg(Tos)GlyOH, 0.92 g of HOBt, and 1.2 g of WSC•HCl were added to this solution under the ice-cooling condition, and its pH value was adjusted to 6 with use of N-methylmorpholine. After removal of DMF by fractionation under a reduced pressure, the residue was dissolved in ethyl acetate, and this ethyl acetate layer was washed with water, 1N HCl, water, 5% NaHCO$_3$, and water, in the order as mentioned, followed by drying the residual substance with use of anhydrous sodium sulfate. Then, the solvent was removed by fractionation under a reduced pressure, thereby obtaining 4.4 g (a rate of yield of 75%) of the intended product.

The amino acid analyses indicated: Asx 1.84, Gly 2.17, Ala 0.96, Arg 1.04, and Pro 0.97.

(5) Synthesis of BocGlyArg(Tos)GlyAsp(OcHex)AsnProAlaOH

4.4 g (75%) of BocGlyArg(Tos)GlyAsp(OcHex)AsnProAlaOBzl was dissolved in a mixed solvent consisting of 50 mℓ of methanol, 0.5 mℓ of water and 0.5 mℓ of acetic acid, to which 1 g of 5% Pd-C. and then hydrogen was caused to pass through the mixture solution for five hours. After filtration, the filtered liquid was fractionated under a reduced pressure. Then, diethyl ether was added to the residue to solidify the same, followed by filtering and drying the same, to thereby obtain 4.5 g (4.1 mmol) of the intended product.

(6) Synthesis of ⌐GlyArg(Tos)GlyAsp(OcHex)AsnProAla⌐

3 g (2.7 mmol) of BocGlyArg(Tos)GlyAsp(OcHex)AsnProAlaOH was dissolved in 10 mℓ of DMF, to which 0.6 g of HOSu and 1 g of WSC•HCl were added under the ice-cooling condition, and the batch was agitated overnight. After removing DMF by fractionation under a reduced pressure, water was added to the residual substance to solidify the same. Then, the solidified substance was sufficiently washed with water and collected by filtration to separate it. After it was dried under a reduced pressure, 15 mℓ of TFA was added, and the mixture was agitated for 15 minutes at room temperature. After removing TFA by fractionation under a reduced pressure, diethyl ether was added to the residual substance to solidify the same. Then, the solidified substance was separated by filtration and dried. The substance was further dissolved in 30 mℓ of DMF, and the solution was dripped in 30 minutes into 1.5 ℓ of pyridine, which had been heated to 53°C, and the batch was agitated for nine hours at 53°C, and overnight at room temperature. After removing pyridine by fractionation under a reduced pressure, the substance was sufficiently washed with water, and then dried, whereby 1.05 g (63%) of the intended product was obtained.

The amino acid analyses indicated: Asx 2.09, Gly 2.6, Ala 1.06, Arg 1.21, and Pro 0.98.

(7) Synthesis of ⌐GlyArgGlyAspAsnProAla⌐

5 mℓ of anisole and 2 mℓ of tricresol were added to 1.03 g of

⌐GlyArg(Tos)GlyAsp(OxHex)AsnProAla⌐ ,

to which 60 mℓ of HF was added, and the batch was reacted for one hour at a temperature of 0°C. After removing HF by fractionation under a reduced pressure, diethyl ether was added to the reaction product to solidify the same, of which precipitate was sufficiently washed with diethyl ether. This precipitate was dissolved into 10% acetic acid, followed by subjecting it to the freeze-drying, whereby 0.98 g of crude peptide was obtained. This crude peptide was refined under the same conditions as in Example 1 (13), to thereby obtain 130 mg of the refined product.

(8) Synthesis of GlyArgGlyAspAsnProAla (synthesis of comparative peptide)

2 mℓ of anisole and 2 mℓ of cresol were added to 1.3 g of BocGlyArg(Tos)GlyAsp(OcHex)-AsnProAlaOBzl, to which 60 mℓ of HF was added, and the batch was reacted for one hour at a temperature of 0°C. After removing HF by fractionation under a reduced pressure, diethyl ether was added to the reaction product to solidify the same, the precipitate of which was sufficiently washed with diethyl ether. The deposited white powder was dissolved into 10% acetic acid, after which it was subjected to freeze-drying, to thereby obtain 0.99 g of crude peptide. This crude peptide was refined under the same conditions as in Example 1 (13), whereby 64 mg of the refined product was obtained.

EXAMPLE 4

A. Synthesis of cyclic peptide ( ┌─R G D S P A V T G─┘ )

(1) Synthesis of BocValThr(Bzl)GlyArg(Tos)GlyOH

2.9 g (2.86 mmol) of BocValThr(Bzl)GlyArg(Tos)GlyOBzl was dissolved in 5 mℓ of methanol, to which 4.3 mℓ of 1N NaOH was dripped, and the mixture was agitated for one hour. This mixture solution was concentrated under a reduced pressure by adding 5 mℓ of 1N HCl, and the concentrate was extracted with 50 ml of ethyl acetate. The ethyl acetate layer was washed twice with 50 mℓ of water, and then dried with use of anhydrous sodium sulfate, followed by removing the solvent by fractionation under a reduced pressure. As the result, 2.3 g (2.8 mmol) of white powder was obtained.

(2) Synthesis of BocValThr(Bzl)GlyArg(Tos)GlyAsp(OBzl)Ser(Bzl)ProAlaOPac

1.63 g (2 mmol) of BocValThr(Bzl)GlyArg(Tos)GlyOH and 1.5 g (1.9 mmol) of $H_2$NAsp(OBzl)Ser(Bzl)-ProAlaOPac were dissolved in 10 mℓ of DMF, to which 0.3 g of HOBt and 0.5 g of WSC•HCl were added, and the batch was agitated overnight. After removing the solvent by fractionation under a reduced pressure, water was added to the reaction product to solidify the same, followed by collecting the same by filtration. Thereafter, the solidified substance was washed with water, 1N HCl, water, 5% NaHCO$_3$, and water, in the order as mentioned. This substance was dried in the vacuum, to obtain 2.66 g (1.79 mmol, 94%) of the intended product.

The amino acid analyses indicated: Asp 0.98, Thr 0.70, Ser 0.82, Gly 1.81, Ala 1. , Val 0.85, Arg 0.71, and Pro 0.91.

(3) Synthesis of BocValThr(Bzl)GlyArg(Tos)GlyAsp(OBzl)Ser(Bzl)ProAlaOH

1.8 g (1.2 mmol) of BocValThr(Bzl)GlyArg(Tos)GlyAsp(OBzl)Ser(Bzl)ProAlaOPac was dissolved in 90% acetic acid, to which 4.5 g of zinc powder was added, and the batch was agitated for one hour. After filtration, the filtered liquid was solidified under a reduced pressure. After adding 1N HCl to this solid phase, it was extracted with chloroform and organic phase was washed with water. After concentration of this organic phase at a reduced pressure, diethyl ether was added to it, and it was kept at a temperature of -20°C. White powder as deposited was collected by filtration and dried to thereby obtain 1.59 g (1.15 mmol, 96%) of the intended product.

## (4) Synthesis of

$$\text{—ValThr(Bzl)GlyArg(Tos)GlyAsp(OBzl)Ser(Bzl)ProAla—}$$

1.4 g (1 mmol) of BocValThr(Bzl)GlyArg(Tos)GlyAsp(OBzl)Ser(Bzl)ProAlaOH was dissolved in 5 mℓ of DMF, to which 0.15 g of HOSu and 0.25 g of WSC•HCl were added under the ice-cooling condition. Further, N-methylmorpholine was added to this mixture solution to adjust its pH value to 6, and the batch was agitated overnight. White powder produced by adding water was extracted with use of ethyl acetate, and concentrated under a reduced pressure. White powder was obtained by adding diethyl ether, which was then washed with water and dried. To this dried powder, 20 mℓ of TFA was added, and the mixture was agitated for 20 minutes at room temperature, followed by eliminating TFA under a reduced pressure. Then, this reaction product was dissolved in 20 mℓ of DMF, and the solution was dripped into pyridine 11 kept at a temperature of 55°C. The batch was reacted for five hours at 55°C and then overnight at 30°C. Thereafter, the reaction product was concentrated under a reduced pressure. White powder resulted from addition of diethyl ether to the residue was collected by filtration and dried, whereby 1.12 g (89%) of the intended product was obtained.

## (5) Synthesis and refining of

$$\text{—ValThrGlyArgGlyAspSerProAla—}$$

0.74 g of crude product was obtained by use of 1.12 g of product obtained in (4) above, and by deprotecting it with use of HF in accordance with the method same as in Example 1 (13), and then this product was further refined under the same conditions as in Example 1 (13) above, to thereby obtain 185 mg of refined cyclic peptide.

The amino acid analyses indicated: Asp 1.04, Thr 0.88, Ser 0.89, Gly 2, Ala 1.0, Val 0.97, Arg 1.0, and Pro 0.93.

(6) Synthesis of linear peptide (ValThrGlyArgGlyAspSerProAla)

The product of (3) above was synthesized in the same manner as in the above (1) through (3), 1.1 g of which was de-protected with use of HF in the same manner as in Example 1 (13), thereby obtaining 0.7 g of crude product, which was further refined in the same manner as mentioned in the foregoing, to obtain 140 mg of linear peptide.

The amino acid analyses indicated: Asp 1.0, Thr 0.80, Ser 0.80, Gly 2, Ala 1.05, Val 0.96, Arg 0.93, and Pro 1.13.

EXAMPLE 5

A. Measurement of cell-adhesion inhibiting activity

(1) Activity of synthesized cyclic peptide to inhibit cells to adhere to fibronectine or vitronectine was measured.

Fibronectine or vitronectine was diluted to 1.0 $\mu$l/mℓ and 2.0 $\mu$l/mℓ with PBS⁻ ($NaH_2PO_4$ 0.005 M + NaCl 0.075 M). 0.5 mℓ of the diluted liquid was put into 24 wells of a plastic plate, and kept at a temperature of 37°C for four hours to thereby coat the liquid on the plate. Subsequently, in order to prevent the cells from their non-peculiar adhesion to the plastic plate, 1% BSA (bovine serum albumin) was added to it and kept at a temperature of 37°C for one hour. After this, the coated plastic plate was washed with PBS⁻ and sufficiently drained water. Then, 0.25 mℓ of peptide solution which had been diluted with D-MEM culture medium (a product of GIBCO) was placed on this coated palstic plate, to which 0.25 ml of suspension of human flat epithelium cancer cells A431 was added and kept at a temperature of 37°C for one hour, to thereby adhere the cells. The thus adhered cells were then washed with D-MEM culture medium for three times to eliminate non-adhered cells, after which those adhered cells were stripped off with EDTA trypsin solution (0.025% + 0.025%) and they were dyed with Tripan Blue for measuring the number of cells. The results are shown in Table 1 below.

Table 1

Inhibition of Cell-Adhesion onto Fibronectine

(cells/well)

| Peptide/ Concentration | 0 | 0.25 | 0.5 | 1.0 | 1.5 mg/mℓ |
|---|---|---|---|---|---|
| RGD | 152 | – | 162 | – | 83 |
| GRGDSPA | 152 | – | 175 | 126 | 111 |
| ⌐GRGDSPA⌐ | 152 | 57 | 26 | 22 | 13 |

Inhibition of Cell-Adhesion onto Vibronectine

(cells/well)

| Peptide/ Concentration | 0 | 10 | 50 | 100 | 300 | 500 mg/mℓ |
|---|---|---|---|---|---|---|
| RGD | 248 | – | 133 | 104 | 73 | – |
| GRGDSPA | 248 | 158 | 115 | 86 | 68 | 60 |
| ⌐GRGDSPA⌐ | 248 | 58 | 45 | 49 | 33 | 50 |

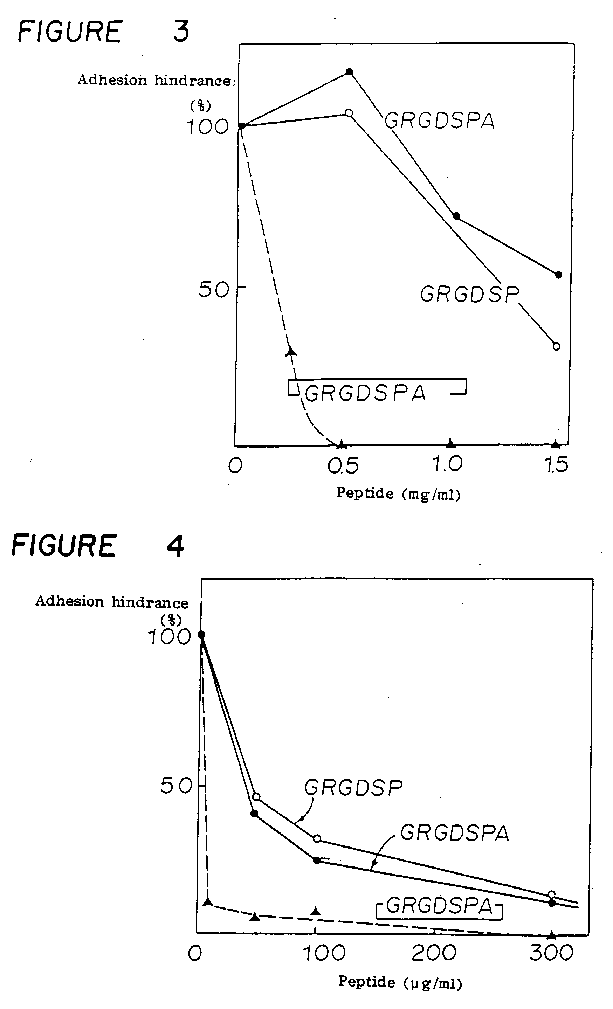

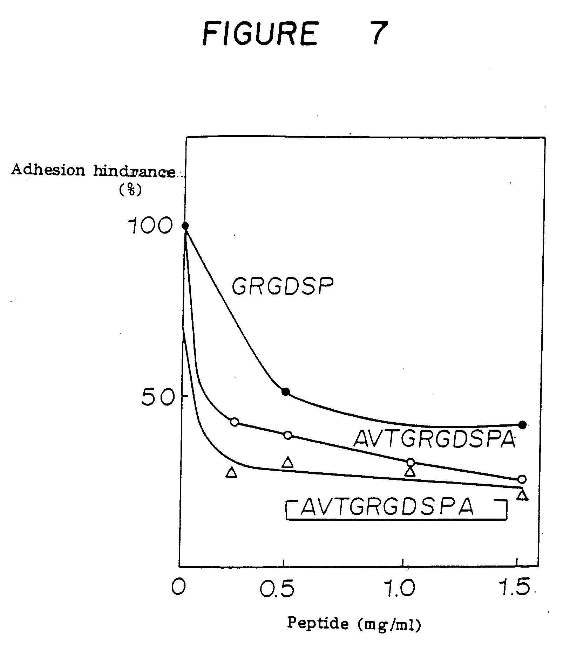

The above results of measurement are shown in the graphical representation of Figures 3 and 4, expressed in terms of percent inhibition of adhesion, wherein Figure 4 indicates the test results with the fibronectine coat, while Figure 5 is those with the vitronectine coat. (2) The same test for inhibition activity as mentioned above was effected on cyclic peptide which was synthesized in Example 2 above (using fibronectine alone). The results are shown in Table 2 below and Figure 7.

24

Table 2

Number of Cells Adhered (cells/well)

| Peptide concentration | 0 | 0.25 | 0.5 | 1.0 | 1.5 mg/ml |
|---|---|---|---|---|---|
| GRGDSP | 375 | – | 227 | – | 198 |
| AVTGRGDSPA | 375 | 204 | 188 | 169 | 193 |
| ⌐AVTGRGDSPA⌐ (cyclic) | 375 | 163 | 168 | 162 | 141 |

B. Activity Test for Inhibiting Blood Platelet Aggregation

In vitro blood platelet aggregation inhibiting activity of a drug to be tested was assayed with use of human blood plasma rich in blood platelet. 3.8% sodium citrate was added to human blood at a ratio of 1/9, and the mixture was centrifuged (1,000 rpm, 10 minutes). The upper layer was taken as the blood plasma rich in blood platelet (PRP). 25 $\mu$l of the drug to be tested was added to 200 $\mu$l of PRP, followed by incubation at 37°C for three minutes. Thereafter, 25 $\mu$l of 20-50 $\mu$M of ADP solution, or 50-200 $\mu$g/ml of collagen solution or thorombin solution was added to this testing drug to measure transmittance of the solution by means of an aggregometer, for the state of aggregation.

Aggregation inhibiting ratio (1 - T/T$_0$) x 100 (%)

T$_0$:    transmittance when no testing drug was added
T:    transmittance when testing drug was added

Table Inhibition of Blood Platelet Aggregation

| | IC$_{50}$ ($\mu$g/ml) | | |
|---|---|---|---|
| | In case of ADP stimulation | In case of collagen stimulation | In case of thrombin stimulation |
| GRGDSPA | 150 | 200 | 1000 |
| ⌐GRGDSPA⌐ (cyclic) | 2.0 | 3.5 | 7.0 |
| ⌐VTGRGDSPA⌐ (cyclic) | 15 | 25 | – |
| ⌐AVTGRGDSPA⌐ (cyclic) | 80 | 100 | – |
| ⌐GRGDNPA⌐ (cyclic) | 2.5 | 6.6 | – |

C. Activity Test for Inhibiting Transfer of Cancer

Effect of

```
 _____
|                |
|                |
 ‾‾‾‾‾‾GRGDSPA‾‾‾‾
```

on the transfer of B16 melanoma cells to the lung of a mouse was examined. $2 \times 10^5$ of B16 melanoma cells were injected into the tail veins of a group of 6 to 10 C57BL mice. After three weeks from the injection, their lungs were taken out, and the number of transferred nodes was counted. Peptide to be tested was dosed simultaneously with injection of the cells. The results are shown in the following Table.

|  | Dosage | Ratio of Inhibition of transfer to lung |
|---|---|---|
| GRGDSPA | 1 mg | 42% |
| GRGDSPA (cyclic) | 0.1 mg | 94% |

D. **Effect of ⌐GRGDSPA⌐ on aggregation of blood platelet at the time of surgical operations**

It has been known that, at the time of the surgical operations using artificial heart and lung, there is induced aggregation of blood platelet, whereby the number of blood platelet decreases.

```
 _____
|                |
|                |
 ‾‾‾‾‾‾GRGDSPA‾‾‾‾
```

has revealed its capability of inhibiting such aggregation at its low concentration, hence it has proved to be useful as the blood platelet aggregation inhibitor. The following Table indicates the results of examination of decrease in the number of the blood platelet, when 100 $\mu$g (per 1 kg of weight/minute) of

```
 _____
|                |
|                |
 ‾‾‾‾‾‾GRGDSPA‾‾‾‾
```

was added for the purpose of the surgical operation. As the control, there was added PBS in place of peptide.

EP 0 406 428 B1

| Time after operation | Blood platelet | |
| --- | --- | --- |
| | Peptide (no addition) | ⌐GRGDSPA⌐ added |
| Before operation | 100% | 100% |
| After 10 minutes | 75% | 90% |
| After 60 minutes | 60% | 95% |

**Claims**

1. Peptide derivatives composed of a synthetic cyclic peptide to be represented by the following formula [I], or its salts:

$$\boxed{\text{—Arg-Gly-Asp-R—}} \quad \cdots [I]$$

wherein R denotes an amino acid residual group or an oligo- to polypeptide residual group and wherein all bonds between the amino acids in the ring are peptide bonds.

2. The peptide derivatives according to Claim 1, wherein R is oligo~polypeptide residual group containing 16 or below of amino acid residual groups.

3. The peptide derivatives according to Claim 1, wherein said sysnthetic cyclic peptide is represented by the following formula [II]:

$$\boxed{\text{—Arg-Gly-Asp-R}^1\text{-Gly—}} \quad \cdots [II]$$

(where: $R^1$ denotes an amino acid residual group or oligo~polypeptide residual group).

4. The peptide derivatives according to Claim 1, wherein R is an oligopeptide residual group represented by the following formula [IV]:

$$-\text{Ser(or Asn)-Pro—[—Ala—]—[—R}^3\text{—]—Gly} \quad \cdots [IV]$$

(where: [ ] denotes a residual group which is existent or non-existent, and, when $R^3$ exists, $R^3$ denotes an amino acid residual group or oligo-polypeptide residual group containing 7 or below of amino acid residual groups).

5. A synthetic cyclic peptide to be represented by any of the following formulae (where an amino acid residual group is indicated by a single letter):

27

R—G—D—S—P—A—G

R—G—D—S—P—A—V—T—G

R—G—D—S—P—A—A—V—T—G

R—G—D—N—P—A—G

6. A drug capable of inhibiting adhesion of animal cells, containing therein, as an effective component, the peptide derivatives according to any one of Claims 1 to 5.

7. The drug according to Claim 6, wherein said animal cells are somatic cells of mammals.

8. The drug according to Claim 6, wherein said animal cells are cancer cells.

9. A drug capable of inhibiting aggregation of blood platelets containing therein, as an effective component, the peptide derivatives according to any one of Claims 1 to 5.

**Patentansprüche**

1. Peptidderivate, zusammengesetzt aus einem durch die folgende Formel [I] darzustellenden, synthetischen cyclischen Peptid oder seinen Salzen:

Arg-Gly-Asp-R ··· [I]

worin R eine Aminosäurerest-Gruppe oder eine Oligo- bis Polypeptidrest-Gruppe darstellt und worin alle Bindungen zwischen den Aminosäuren im Ring Peptidbiniungen sind.

2. Peptidderivate nach Anspruch 1, in denen R eine Oligo-Polypeptidrest-Gruppe ist, die 16 oder weniger Aminosäurerest-Gruppen enthält.

3. Peptidderivate nach Anspruch 1, in denen das synthetische cyclische Peptid durch die folgende Formel [II] dargestellt wird:

Arg-Gly-Asp-R$^1$-Gly ··· [II]

(worin: R$^1$ eine Aminosäurerest-Gruppe oder Oligo-Polypeptidrest-Gruppe darstellt).

4.  Peptidderivate nach Anspruch 1, in denen R eine Oligopeptidrest-Gruppe ist, die durch die folgende Formel [IV] dargestellt wird:

-Ser (oder Asn)-Pro-[-Ala-]-[-R³-]-Gly      [IV]

(worin: [ ] eine Restgruppe bedeutet, die vorhanden ist oder nicht vorhanden ist, und, wenn R³ vorhanden ist, R³ eine Aminosäurerest-Gruppe oder Oligo-Polypeptidrest-Gruppe, die 7 oder weniger Aminosäurerest-Gruppen enthält, darstellt).

5.  Synthetisches cyclisches Peptid, das durch irgendeine der folgenden Formeln darzustellen ist (worin eine Aminosäurerest-Gruppe durch einen einzigen Buchstaben angegeben ist):

$$\boxed{\;R\!-\!G\!-\!D\!-\!S\!-\!P\!-\!A\!-\!G\;}$$

$$\boxed{\;R\!-\!G\!-\!D\!-\!S\!-\!P\!-\!A\!-\!V\!-\!T\!-\!G\;}$$

$$\boxed{\;R\!-\!G\!-\!D\!-\!S\!-\!P\!-\!A\!-\!A\!-\!V\!-\!T\!-\!G\;}$$

$$\boxed{\;R\!-\!G\!-\!D\!-\!N\!-\!P\!-\!A\!-\!G\;}$$

6.  Arzneimittel, das in der Lage ist, die Haftung von Tierzellen zu hemmen, das darin als wirksame Komponente die Peptidderivate nach irgendeinem der Ansprüche 1 bis 5 enthält.

7.  Arzneimittel nach Anspruch 6, in dem die Tierzellen somatische Zellen von Säugern sind.

8.  Arzneimittel nach Anspruch 6, in dem die Tierzellen Krebszellen sind.

9.  Arzneimittel, das in der Lage ist, die Aggregation von Blutplättchen zu hemmen, das darin als wirksame Komponente die Peptidderivate nach irgendeinem der Ansprüche 1 bis 5 enthält.

**Revendications**

1.  Dérivés peptidiques composés d'un peptide cyclique de synthèse devant être représenté par la formule [I] suivante, ou ses sels :

$$\boxed{\;Arg\!-\!Gly\!-\!Asp\!-\!R\;} \quad \cdots \;[I]$$

dans laquelle R désigne un groupe résiduel amino acide ou un groupe résiduel oligo- à polypeptide et dans laquelle toutes les liaisons entre les amino acides dans le cycle sont des liaisons peptidiques.

2.  Dérivés peptidiques selon la revendication 1, dans lesquels R représente un groupe résiduel oligo- à polypeptide contenant 16 groupes résiduels amino acide ou moins.

29

3. Dérivés peptidiques selon la revendication 1, dans lesquels ledit peptide cyclique de synthèse est représenté par la formule [II] suivante :

$$\boxed{\text{—Arg–Gly–Asp–}R^1\text{–Gly—}} \qquad \cdots \ [\text{II}]$$

(où : $R^1$ désigne un groupe résiduel amino acide ou un groupe résiduel oligo- à polypeptide).

4. Dérivés peptidiques selon la revendication 1, dans lesquels R représente un groupe résiduel oligopeptide représenté par la formule [IV] suivante :

$$-\text{Ser(or Asn)}-\text{Pro}-[-\text{Ala}-]-[-R^3-]-\text{Gly} \qquad \cdots \ [\text{IV}]$$

(où : [ ] désigne un groupe résiduel qui existe ou n'existe pas, et, lorsque $R^3$ existe, $R^3$ désigne un groupe résiduel amino acide ou un groupe résiduel oligo- à polypeptide contenant 7 groupes résiduels amino acide ou moins).

5. Peptide cyclique de synthèse devant être représenté par n'importe laquelle des formules suivantes (où un groupe résiduel amino acide est indiqué par une simple lettre) :

$$\boxed{\text{—R—G—D—S—P—A—G—}}$$

$$\boxed{\text{—R—G—D—S—P—A—V—T—G—}}$$

$$\boxed{\text{—R—G—D—S—P—A—A—V—T—G—}}$$

$$\boxed{\text{—R—G—D—N—P—A—G—}}$$

6. Médicament capable d'inhiber l'adhérence de cellules animales, contenant en son sein, en tant que composant efficace, les dérivés peptidiques tels que définis à l'une des revendications 1 à 5.

7. Médicament selon la revendication 6, dans lequel lesdites cellules animales sont des cellules somatiques de mammifères.

8. Médicament selon la revendication 6, dans lequel lesdites cellules animales sont des cellules cancéreuses.

9. Médicament capable d'inhiber l'agrégation de plaquettes sanguines contenant en son sein, en tant que composant efficace, les dérivés peptidiques tels que définis à l'une des revendications 1 à 5.

# *FIGURE    1*

GRGDSPA

GDSPAGR

Trypsin treatment

Control

Time (min.)

# *FIGURE    2*

GRGDSPA

GDSPA

GR

Trypsin treatment

Control

Time (min.)

FIGURE  3

FIGURE  4

# FIGURE 5

GDSPA AVTGR

AVTGRGDSPA

0          10          20          30

# FIGURE 6

AVTGRGDSPA

GDSPA

AVTGR

0          10          20          30

FIGURE 7